(19) Europäisches Patentamt
European Patent Office
Office européen des brevets

(11) Publication number: **0 400 842 A1**

(12) **EUROPEAN PATENT APPLICATION**

(21) Application number: **90305347.8**

(22) Date of filing: **17.05.90**

(51) Int. Cl.[5]: **C07D 249/06, C07D 401/04, C07D 403/04, A01N 43/647, C07C 247/16**

(30) Priority: **18.05.89 GB 8911387**

(43) Date of publication of application: **05.12.90 Bulletin 90/49**

(84) Designated Contracting States: **AT BE CH DE DK ES FR GB GR IT LI LU NL SE**

(71) Applicant: **SCHERING AGROCHEMICALS LIMITED**
**Hauxton Cambridge CB2 5HU(GB)**

(72) Inventor: **Willis, Robert John**
**15 Cherry Orchard**
**Fulbourn Cambridge(GB)**
Inventor: **Marlow, Ian David**
**59 Back Road**
**Linton Cambridge(GB)**

(74) Representative: **Waldman, Ralph David et al**
**Schering Agrochemicals Limited Industrial Property Department Chesterford Park Research Station**
**Saffron Walden Essex CB10 1XL(GB)**

(54) **Triazole pesticides.**

(57) Compounds of formula I

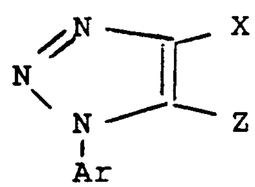

(I)

in which Ar is phenyl, pyridyl or pyrimidinyl, each of which is optionally substituted, and X and Z, which may be the same or different, are as defined in the description, have insecticidal and acaricidal activity. Many compounds are novel, as are certain intermediates.

EP 0 400 842 A1

## Triazole Pesticides

This invention relates to new compounds having insecticidal activity.

The present invention provides an insecticidal or acaricidal composition which comprises a compound of formula I

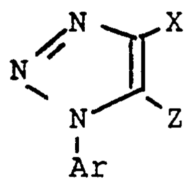

(I)

Ar is phenyl, pyridyl or pyrimidinyl, each of which is optionally substituted;
X and Z, which may be the same or different are hydrogen, halogen, cyano, nitro, carbamoyl, $SOR^1$, $SO_2R^1$, $NR^2R^3$, $SR^4$, $OR^4$, $COOR^5$, $SiR^6R^7R^8$, aryl or optionally substituted alkyl;
Z has the same meaning as X or is amino, with the proviso that X and Z cannot both be hydrogen;
$R^1$ is optionally substituted alkyl;
$R^2$ is optionally substituted alkyl or acyl, or
$R^3$ and $R^4$ may be the same or different and have the same meaning as $R^2$ or are hydrogen, or
$R^2$ and $R^3$ together with the nitrogen to which they are attached, form a 5 to 7 membered ring which can contain other hetero atoms; and
$R^5$ is hydrogen, optionally substituted alkyl or aryl;
$R^6$, $R^7$ and $R^8$, may be the same or different and are optionally substituted alkyl or aryl;
said compound being in admixture with an agriculturally acceptable diluent or carrier.

Many of the compounds of formula I are novel and the invention includes all such novel compounds and especially compounds where X and Z are as defined above and Ar is 4-trifluoromethylphenyl, in which the phenyl group is optionally further substituted.

The invention also includes as intermediates, compounds of formula I, where Z is as defined above, X is amino and Ar is 4-trifluoromethylphenyl, in which the phenyl group is optionally further substituted.

Alkyl and alkoxy groups are preferably of 1 to 4 carbon atoms, especially methyl. Substituents, when present on any alkyl group, include halogen, alkoxy (e.g. of 1 to 4 carbon atoms), hydroxy, optionally substituted alkylthio, nitro, optionally substituted amino, carboxy, alkoxycarbonyl, acyloxy and aryl. Aryl groups are usually phenyl, optionally substituted, e.g. by halogen, alkyl, haloalkyl, alkoxy, alkylthio, haloalkoxy, haloalkylthio or nitro. The term aryl may include heteroaryl groups such as thienyl, furyl or pyridyl. The term "acyl" includes the residue of sulphonic and phosphorus containing acids as well as carboxylic acids. Acyl groups are preferably alkanoyl e.g. of 1 to 4 carbon atoms. Amino groups may be substituted, e.g. by one or two alkyl groups or two substituents can form a ring, e.g. to form a morpholino or piperidino ring.

When $R^2$ and $R^3$ form a ring with the nitrogen to which they are attached, this is generally a morpholine or piperidine ring. This ring can carry another fused ring and/or can be substituted, e.g. by one or more optionally substituted alkyl groups.

A particularly preferred group of compounds are those where Ar is 2,6-dichloro-4-trifluoromethylphenyl The compounds of the invention have insecticidal and acaricidal activity and are particularly useful in combating a variety of economically important insects, and acarids including animal ectoparasites, e.g. Lepidoptera, including Spodoptera littoralis, Heliothis armigera, and Pieris brassicae; Diptera, including Musca domestica, Ceratitis capitata, Erioischia brassicae, Lucilia sericata and Aedes aegypti; Homoptera, including aphids such as Megoura viciae and Nilaparvata lugens; Coleoptera, including Phaedon cochleariae, Anthonomus grandis and corn rootworms (Diabrotica spp. eg. Diabrotica undecimpunctata); Orthoptera, including Blattella germanica; ticks, e.g. Boophilus microplus and lice, including Damalinia bovis and Linognathus vituli.

The invention thus also includes a method of combating insect and acarid pests at a locus infested or liable to be infested therewith, which comprises applying to the locus a compound of formula I.

More than one compound of the invention can, of course, be used in carrying out the invention. In addition one or more additional pesticides for example compounds known to possess herbicidal, fungicidal, insecticidal, acaricidal or nematicidal properties can be used in association with the compounds of formula I. Alternatively the compounds of the invention can be used in sequence with the other pesticides.

The diluent or carrier in the composition of the invention can be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty alcohols, fatty acid amides or fatty alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetramethyl-5-decyne-4,7-diol, or ethoxylated acetylenic glycols. Examples of a cationic surface-active agent include, for instance, an aliphatic mono-, di-, or polyamine as an acetate, naphthenate or oleate; an oxygen-containing amine such a amine oxide or polyoxyethylene alkylamine; an amide-linked amine prepared by the condensation of a carboxylic acid with a di- or polyamine; or a quaternary ammonium salt.

The compositions of the invention can take any form known in the art for the formulation of insecticidal compounds, for example, a solution, a dispersion, an aqueous emulsion, a dusting powder, a seed dressing, a fumigant, a smoke, a dispersible powder, an emulsifiable concentrate, granules, pour-ons, ear-tags or baits. Moreover it can be in a suitable form for direct application or as a concentrate or primary composition which requires dilution with a suitable quantity of water or other diluent before application.

As a dispersion, the composition comprises a compound of the invention dispersed in a liquid medium, preferably water. It is often convenient to supply the consumer with a primary composition which can be diluted with water to form a dispersion having the desired concentration. The primary composition can be provided in any one of the following forms. It can be a dispersible solution which comprises a compound of the invention dissolved in a water-miscible solvent with the addition of a dispersing agent. A further alternative comprises a compound of the invention in the form of a finely ground powder in association with a dispersing agent and intimately mixed with water to give a paste or cream which can if desired be added to an emulsion of oil in water to give a dispersion of active ingredient in an aqueous oil emulsion.

An emulsifiable concentrate comprises a compound of the invention dissolved in a water-immiscible solvent together with an emulsifying agent and which is formed into an emulsion on mixing with water.

A dusting powder comprises a compound of the invention intimately mixed with a solid pulverulent diluent, for example, kaolin.

A granular solid comprises a compound of the invention associated with similar diluents to those which may be employed in dusting powders, but the mixture is granulated by known methods. Alternatively it comprises the active ingredient adsorbed or absorbed on a pre-granular diluent, for example, Fuller's earth, attapulgite or limestone grit.

A wettable powder usually comprises the active ingredient in admixture with a suitable surfactant and an inert powder diluent such as china clay.

Another suitable concentrate, particularly when the product is a solid, is a flowable suspension concentrate which is formed by grinding the compound with water, a wetting agent and a suspending agent.

Baits can include an attractant and may comprise a protein hydrolysate e.g. for the control of fruit flies, sugar e.g. for the control of adult Musca spp. or corn cob e.g. for the control of cockroaches.

The concentration of the active ingredient in the composition of the present invention is preferably within the range of 1 to 30 per cent by weight, especially 5 to 30 per cent by weight. In a primary composition the amount of active ingredient can vary widely and can be, for example, from 5 to 95 per cent by weight of the composition.

The compounds of the invention may be prepared by a variety of methods known in the art, for example by reacting a compound of formula II

$ArN_3$ (II)

with

a) a compound of formula III

$XCH_2Y$ (III)

where Y is CN, under basic conditions, to give a compound of formula I, in which Z is $NH_2$;

b) a compound of formula IV

X-C≡C-Z (IV)

c) a compound of formula V

X-CH = CH-$NR^2R^3$ (V)

to give a compound of formula I, in which Z is H; or

d) a compound of formula VI

ArSO- C = CH-$NR^2R^3$ (VI)

X

in which Ar is an optionally substituted phenyl group, especially p-tolyl, to give a compound in which Z is hydrogen or $NR^2R^3$, depending on the reaction conditions, and optionally, where possible, modifying X and Z groups in known manner to give other desired X and Z groups.

The processes may give a mixture of regioisomers, which can be separated, if desired, by conventional means and usually identified by nmr experiments.

The compound of formula II can be prepared in known manner from the corresponding amine of formula $ArNH_2$. The compound of formula II, where Ar is 2,6-dichloro-4-trifluoromethylphenyl, is novel and forms part of the invention as a novel intermediate. Compounds of formulae III, IV, V and VI are either known or can be obtained in known manner.

Thio groups can be converted to sulphinyl or sulphonyl groups by oxidation, e.g. using a suitable per acid.

The invention is illustrated in the following examples. Structures of isolated novel compounds were confirmed by elemental and/or other appropriate analyses. Temperatures are in °C.

Example 1 (route a)

A solution of sodium nitrite (3.2 g) in concentrated sulphuric acid (22 ml) was added dropwise with cooling to a solution of 2,6-dichloro-4-trifluoromethylaniline (9.3 g) in glacial acetic acid (48 ml). The mixture was stirred at room temperature for ½ hour and then cooled to 0-5°. A solution of sodium azide (2.6 g) in the minimum volume of water was added dropwise with cooling and the mixture stirred at 0-5° for 1 hour and then at room temperature overnight. The mixture was poured into ice/water, extracted with dichloromethane and the extract worked up in conventional manner to give 1-azido-2,6-dichloro-4-trifluoromethylbenzene, as a red oil. A solution of this oil (3.9 g) in methanol (10 ml) was added dropwise with stirring to a solution prepared by adding methylsulphonylacetonitrile (1.82 g) to sodium (0.35 g) in methanol (30 ml). The mixture was stirred at room temperature overnight, heated under reflux for 3 hours, cooled and poured into water. It was then extracted with ethyl acetate and the extracts worked up in conventional manner and the product purified by column chromatography to give 5-amino-1-(2,6-dichloro-4-trifluoromethylphenyl)-4-methylsulphonyl-1H-1,2,3-triazole, m.p. 134-5°. (Compound 1)

Example 2 (route b)

A mixture of 1-azido-2,6-dichloro-4-trifluoromethylbenzene (5.12 g) and ethynyltrimethylsilane (2.7 ml) in toluene (10 ml) was stirred under nitrogen for 4 days. It was then heated to 105° to remove excess ethynyltrimethylsilane, following which solvent was evaporated under reduced pressure. The residue was triturated with light petroleum (bp 30-40°) and the resulting solid collected and recrystallised from light petroleum (bp 30-40°) to give 1-(2,6-dichloro-4-trifluoromethylphenyl)-4-trimethylsilyl-1H-1,2,3-triazole, m.p. 124-6°. (Compound 2) The regiochemistry was confirmed by NOE nmr experiments.

Example 3 (route c)

A mixture of 1-azido-2,6-dichloro-4-trifluoromethylbenzene (16.2 g) and N-(2-nitroethenyl)morpholine (10.0 g) in ethanol (250 ml) was heated under reflux for 4 days. The resulting solid was collected and dissolved in dichloromethane and the solution passed through a silica gel column to give 1-(2,6-dichloro-4-trifluoromethylphenyl)-4-nitro-1H-1,2,3-triazole, m.p. 187-9°. (Compound 3)

Example 4

In a similar manner to one of the previous Examples, the following compounds of formula I, in which Ar is 2,6-dichloro-4-trifluoromethylphenyl were obtained

| Compound No | X | Z | Route | m.p. (°) |
|---|---|---|---|---|
| 4 | $SO_2Pr^i$ | $NH_2$ | a | 138.5-141 |
| 5 | $CH_2SMe$ | H | b | 85-87 |
| 6 | H | $CH_2SMe$ | b | 86-88 |
| 7 | $CH_2SEt$ | H | b | 62-64 |
| 8 | COOMe | SMe | b | 128-130 |
| 9 | $Bu^t$ | H | b | 99-101 |
| 10 | $SiMe_3$ | $SiMe_3$ | b | 129-131 |
| 11 | $SiMe_3$ | Me | b | 103-105 |
| 12 | H | COOMe | b | 118-120 |
| 13 | H | $CH_2OMe$ | b | 88-90 |
| 14 | H (or Ph) | Ph (or H) | b | 119-122 |
| 15 | Ph (or H) | H (or Ph) | b | 166-9 |
| 16 | $CH_2SiMe_3$ | H | b | 89-91 |
| 17 | $CH_2SPr^i$ | H | b | 75-76 |
| Note: compounds 14 and 15 are isomers - the configuration of each isomer could not be identified. | | | | |

Example 5

A mixture of 1-azido-2,6-dichloro-4-trifluoromethylbenzene (1.25 g), N,N-dimethyl-2-methylsulphonyl-2-(p-tolylsulphinyl)vinylamine (1.43 g) and methanol (20 ml) was heated under reflux for 60 hours. The mixture was evaporated under reduced pressure and the residue triturated with light petroleum (bp 40-60° ). The solid residue was triturated with boiling diisopropyl ether and the product recrystallised from diisopropyl ether to give 1-(2,6-dichloro-4-trifluoromethylphenyl)-4-methylsulphonyl-1H-1,2,3-triazole, m.p. 134-5° . (Compound 18)

Example 6

A solution of compound 1 (1.87 g) and 2,5-dimethoxytetrahydrofuran (1.98 g) in glacial acetic acid (40 ml), under nitrogen, was heated under reflux, overnight. After cooling, the mixture was evaporated under reduced pressure and the residue dissolved in dichloromethane. The solution was washed with water, saturated aqueous sodium hydrogen carbonate and water, dried and evaporated under reduced pressure. The residue was purified by column chromatography to give 1-(2,6-dichloro-4-trifluoromethylphenyl)-5-(1-pyrrolyl)-4-methylsulphonyl-1H-1,2,3-triazole, m.p. 125-9° . (Compound 19)

Example 7

To a stirred solution of compound 1 (0.75 g) in bromoform (10.4 g) was added t-butyl nitrite and the mixture stirred overnight. It was evaporated under reduced pressure and the residue dissolved in dichloromethane. The solution was washed with saturated aqueous sodium hydrogen carbonate, aqueous sodium thiosulphate and water, dried and evaporated under reduced pressure. The residue was purified by column chromatography to give 5-bromo-1-(2,6-dichloro-4-trifluoromethylphenyl)-4-methylsulphonyl-1H-1,2,3-triazole, m.p. 128-130° . (Compound 20)

Test Example

1) Ticks (Boophilus microplus) - larval test

Filter papers (9 cm diameter) were impregnated with 1 ml aliquots of acetone solutions of test compound at various concentrations. The papers were allowed to dry and then folded into envelopes in which cattle tick larvae. (Boophilus microplus) were enclosed and held at 25° C and 80% relative humidity for 48 hours. The percentage mortality of tick larvae was then recorded and compared with controls. The controls gave less than 5% mortality, whereas compounds 1-5, 7, 9, 16 and 18-20 had an $LC_{50}$ of less than 300 ppm.

(2) Sheep blowfly (Lucilia sericata)

1 ml aliquots of an acetone solution containing test compound at various concentrations were applied to cotton wool dental rolls (1 cm x 2 cm), contained in glass vials 2 cm diameter x 5 cm long. After drying, the treated materials were then impregnated with 1 ml of nutrient solution, infested with first instar larvae of sheep blow fly (Lucilia sericata), closed by a cotton wool plug and held at 25° C and 80% relative humidity for 24 hours. For the controls the mortality was <5% whereas compounds 1, 2, 4-11, 13, 14 and 17-20 had an $LC_{50}$ of less than 300 ppm.

3) House fly (Musca domestica)

Aliquots of acetone solutions of test compounds at various concentrations were applied to filter papers (9 cm diameter) placed in the bottom of petri dishes (9 cm diameter) closed by glass lids. After evaporation of solvent, the treated surfaces, together with controls treated with acetone alone. were then infested with adult houseflies, (Musca domestica) and held at 22° C and 80% R.H. for 24 hours.The percentage mortality of the insects was then recorded. Less than 5% mortality resulted in the controls whereas compounds 1, 2, 7-12, 16 and 18-20 had an $LC_{50}$ of less than 300 $mg/m^2$

4. Brown rice-hoppers (Nilaparvata lugens Stal)

Rice seedlings (Oryzae sativa L.) in the two leaf stage (about 10 per polystyrene pot of size 6.5 x 6.5 cm) were either untreated or dipped until dripping wet, with an aqueous preparation of the test compound. After drying the sprayed leaves, a transparent cylinder was placed over each pot and through an opening, about 30 brown rice-hoppers (Nilaparvata lugens) in the 4-5 stage, anaesthetised with carbon dioxide, were introduced into each pot. After closing the opening with a fine mesh screen, the pots were kept for 2 days at 28° C and 16 hours/day of light in the glasshouse, the amount of dead hoppers was determined. The percentage mortality was then estimated and the activity calculated using Abbott's method in comparison with the untreated controls.

Compounds 2, 4, 9, 11 and 15 had an $Lc_{50}$ of less than 300 ppm.

## Claims

1) An insecticidal or acaricidal composition which comprises a compound of formula I

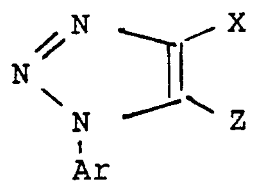

(I)

Ar is phenyl, pyridyl or pyrimidinyl, each of which is optionally substituted;
X and Z, which may be the same or different are hydrogen, halogen, cyano, nitro, carbamoyl, $SOR^1$, $SO_2R^1$, $NR^2R^3$, $SR^4$, $OR^4$, $COOR^5$, $SiR^6R^7R^8$, aryl or optionally substituted alkyl;
Z has the same meaning as X or is amino, with the proviso that X and Z cannot both be hydrogen;

$R^1$ is optionally substituted alkyl;
$R^2$ is optionally substituted alkyl or acyl, or
$R^3$ and $R^4$ may be the same or different and have the same meaning as $R^2$ or are hydrogen, or
$R^2$ and $R^3$ together with the nitrogen to which they are attached, form a 5 to 7 membered ring which can contain other hetero atoms; and
$R^5$ is hydrogen, optionally substituted alkyl or aryl;
$R^6$, $R^7$ and $R^8$, may be the same or different and are optionally substituted alkyl or aryl;
said compound being in admixture with an agriculturally acceptable diluent or carrier.

2. Compounds of formula I as disclosed in claim 1, where X and Z are as defined in claim 1 and Ar is 4-trifluoromethylphenyl, in which the phenyl group is optionally further substituted.

3. Compounds of formula I as disclosed in claim 1, useful as intermediates, in which Z and Ar are as defined in claim 2 and X is amino.

4. A method of combating insects and acarids which comprises applying to the insect or acarid or their locus, a compound disclosed in claim 1 or 2.

5. As a novel intermediate, 1-azido-2,6-dichloro-4-trifluoromethylbenzene.

European Patent Office

# EUROPEAN SEARCH REPORT

Application Number

EP 90 30 5347

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS vol. 98, no. 7, 14 February 1983, page 664, abstract no. 53790n, Columbus, Ohio, US; A. DA SETTIMO et al.: "1,2,3-Triazole derivatives of aryloxyalkanoic acids" & Farmaco, Ed. Sci. 1982, vol. 37, no. 11, pages 728-739 | 1 | C 07 D 249/06<br>C 07 D 401/04<br>C 07 D 403/04<br>A 01 N 43/647<br>C 07 C 247/16 |
| X | US-A-4 610 994 (P.K. KADABA)<br>* claims 1,13 * | 2 | |
| X | CHEMICAL ABSTRACTS vol. 88, no. 9, 27 February 1987, pages 123, abstract no. 59288v, Columbus, Ohio, US; V. MESSORI et al.: "1,2,3-Triazole derivatives with herbicidal activity" & Chim. Ind. (Milan) 1977, vol. 59, no. 6, pages 438-440 in connection with CHEMICAL ABSTRACTS, vol. 88, 1978, Chemical Substance Index, Pp-Z, page 5164CS, left-hand column, lines 111-113 | 2 | **TECHNICAL FIELDS SEARCHED (Int. Cl.5)** |
| X | CHEMICAL ABSTRACTS vol. 108, no. 5, 1 February 1988, page 628, abstract no. 37738w, Columbus, Ohio, US; P.K. KADABA: "Triazolines. 14. 1,2,3-Triazolines and triazoles. A new class of anticonvulsants. Drug design and structure-activity relationships" & J. Med. Chem. 1988, vol. 31, no. 1, pages 196-203 in connection with CHEMICAL ABSTRACTS, vol. 108, 1988, Chemical Substance Index, Pheo-Q, page 8020CS, right-hand column, lines 72,73<br>-/- | 2 | A 01 N 43/00<br>C 07 C 247/00<br>C 07 D 249/00<br>C 07 D 401/00<br>C 07 D 403/00 |

The present search report has been drawn up for all claims

| Place of search | Date of completion of the search | Examiner |
|---|---|---|
| BERLIN | 30-08-1990 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 90 30 5347

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | CHEMICAL ABSTRACTS vol. 111, no. 21, 20 November 1989, page 765, abstract no. 194677e, Columbus, Ohio, US; H. WAMHOFF et al.: "Heterocyclic beta-enamino esters. 51. Carbon-13 NMR studies of the Dimroth rearrangement of 1,2,3-triazoles" & Chem.-Ztg. 1989, vol. 113, no. 1, pages 11-15 (cat. X) in connection with CHEMICAL ABSTRACTS, vol. 111, 1989, Chemical Substance Index, Si-Z, page 10412CS, right-hand column, keyword "1H-1,2,3-Triazole-4-carboxylic acid"; page 10413CS, left-hand column, lines 2-5 --- | 2 | |
| X | US-A-3 291 793 (H.U. DAENIKER) * example 1, starting material; example 4, starting material * --- | 3 | |
| X | CHEMICAL ABSTRACTS vol. 66, no. 3, 16 January 1967, page 1055, abstra ct no. 10887k, Columbus, Ohio, US; G. REMBARZ et al.: "Reaction of phenyl azide with omega-nitrostyrenes" & J. Prakt. Chem., vol. 33, nos. 3,4, pages 199-205 --- | 3 | TECHNICAL FIELDS SEARCHED (Int. Cl.5) |
| X | CHEMICAL ABSTRACTS vol. 70, no. 9, 3 March 1969, page 345, abstract no. 37725m, Columbus, Ohio, US; D. POCAR et al.: "Enamines. XXXI. 4-Nitropyrazoles and 4-nitro-v-triazoles" & Gazz. Chim. Ital. 1968, vol. 98, nos. 8,9, pages 949-957 --- -/- | 3 | |

The present search report has been drawn up for all claims

| Place of search | Date of completion of the search | Examiner |
|---|---|---|
| BERLIN | 30-08-1990 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 90 30 5347

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DE-A-1 542 769 (FARBWERKE HOECHST AG)<br>* claims 1,4; examples 1-3 *<br>--- | 1,4 | |
| Y | US-A-4 775 762 (I.L. KNOX et al.)<br>* examples 6-10 *<br>--- | 1,4 | |
| Y | GB-A-2 102 677 (SANDOZ LTD.)<br>* claims 1-3; page 7, example Z 26; page 3, lines 25-33; page 4, example 1 * | 1,4 | |
| A | <br>--- | 5 | |
| A | US-A-3 579 531 (P. SCHEINER)<br>* column 1, line 48 - column 2, line 13 *<br>--- | 2,5 | |
| A | US-A-4 474 599 (R.B. ROGERS et al.)<br>* examples 1-4 *<br>--- | 2,5 | |
| A | GB-A-1 459 060 (SOCIETA ITALIANA RESINE S.I.R. S.P.A.)<br>* claim 1 *<br>--- | 2 | TECHNICAL FIELDS SEARCHED (Int. Cl.5) |
| A | GB-A-2 070 607 (SANDOZ LTD.)<br>* claim 13 *<br>--- | 2 | |
| A,P | EP-A-0 350 237 (SCHERING AGROCHEMICALS LTD.)<br>* claims 1-4; abstract *<br>----- | 1,2,4 | |

The present search report has been drawn up for all claims

| Place of search | Date of completion of the search | Examiner |
|---|---|---|
| BERLIN | 30-08-1990 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)